# EUROPÄISCHE PATENTSCHRIFT

(11) **EP 1 521 755 B1**
(45) Veröffentlichungstag und Bekanntmachung des Hinweises auf die Patenterteilung: **23.01.2008**
(21) Anmeldenummer: 03735569.0
(22) Anmeldetag: 06.06.2003
(51) Int. Cl.: C07D 487/04, C07D 403/04, C07D 235/26

(54) **NEUE DIKETOPYRROLOPYRROLPIGMENTE**
NOVEL DIKETOPYRROLOPYRROLE PIGMENTS
NOUVEAUX PIGMENTS DU TYPE DICETOPYRROLOPYRROL

(30) Priorität: 10.07.2002 DE 10231105
(43) Veröffentlichungstag der Anmeldung: 13.04.2005
(73) Patentinhaber: Clariant Produkte (Deutschland) GmbH, 65929 Frankfurt am Main (DE)
(72) Erfinder: GRIMM, Felix, W., 65719 Hofheim (DE); METZ, Hans, Joachim, 64285 Darmstadt (DE); WEBER, Joachim, 65929 Frankfurt am Main (DE); WACKER, Andreas, 68165 Mannheim (DE)
(86) Internationale Anmeldenummer: PCT/EP2003/005968
(87) Internationale Veröffentlichungsnummer: WO 2004/007500

(56) Entgegenhaltungen:
- EP-A- 0 094 911
- EP-A- 0 098 808
- EP-A- 0 133 156
- EP-A- 0 755 933

## Beschreibung

Die vorliegende Erfindung betrifft neue 1,4-Diketopyrrol-[3,4-c]-pyrrole, im folgenden Diketopyrrolopyrrole genannt, die wertvolle Pigmente darstellen, sowie deren Herstellung und Verwendung zum Pigmentieren von hochmolekularen Materialien.

In EP-A-0 061 426 wird ein Verfahren zum Färben von hochmolekularem organischen Material in der Masse, gekennzeichnet durch die Verwendung eines Diketopyrrolopyrrols, offenbart.

In EP-A-0 094 911 wird ein Verfahren zur Herstellung von Diketopyrrolopyrrolen offenbart.

Bei der Verwendung von Pigmenten zum Färben von hochmolekularen organischen Materialen werden hohe Anforderungen an die anwendungstechnischen Eigenschaften der Pigmente gestellt, wie hohe Farbstärke, gute Licht- und Wetterechtheit, bei Einsatz in Lacksystemen einwandfreie Überlackierechtheiten, niedrige Viskosität der hochpigmentierten Lackkonzentrate (millbase) und insbesondere im Falle von Metalliclackierungen hohe Transparenz und brillante Farbtöne. Im Falle der Kunststoffeinfärbung wird gute Dispergierbarkeit verlangt, was z.B. in hohen Farbstärken zum Ausdruck kommt. Auch in Drucksystemen sind hohe Farbstärken gefordert. Die Pigmente sollen möglichst universell einsetzbar sein.

Zahlreiche der in den oben genannten Schriften offenbarten Diketopyrrolopyrrole genügen jedoch nicht mehr den heutigen Anforderungen.

Es bestand daher die Aufgabe, neue Diketopyrrolopyrrolpigmente zu finden, die verglichen zu den bislang bekannten Diketopyrrolopyrrolpigmenten überlegene Eigenschaften besitzen.

Es wurde gefunden, dass die Aufgabe überraschenderweise durch nachstehend definierte Diketopyrrolopyrrolpigmente gelöst wird.

Gegenstand der Erfindung sind Diketopyrrolopyrrole der allgemeinen Formel (I), 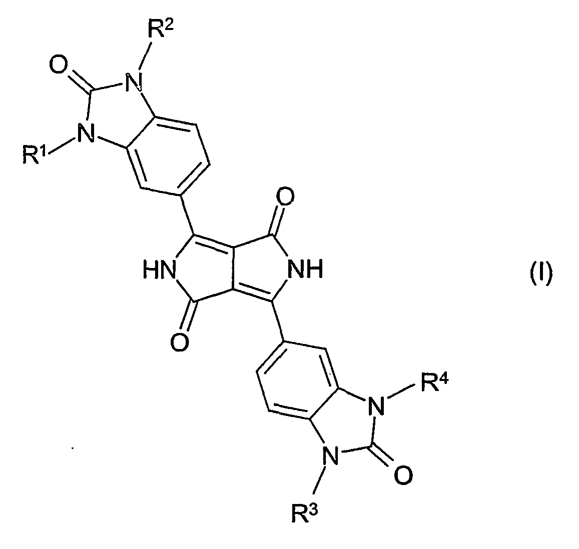 worin
R¹, R², R³ und R⁴ unabhängig voneinander einen C₁-C₄-Alkylrest oder einen substituierten oder unsubstituierten Phenylrest darstellen, wobei der Phenylrest durch 1, 2, 3 oder 4 Substituenten aus der Gruppe C₁-C₄-Alkyl, C₁-C₄-Alkoxy, CN, F, Cl, Br, NO₂, CF₃, S-C₁-C₄-Alkyl, Phenyl oder (C₁-C₂)-Alkylenphenyl substituiert sein kann,
mit der Maßgabe, dass mindestens einer der Reste R¹, R², R³, oder R⁴ einen der genannten substituierten oder unsubstituierten Phenylreste bedeutet.

Gegenstand der vorliegenden Erfindung sind auch Gemische aus zwei oder mehreren, z.B. 2 oder 3, Diketopyrrolopyrrolen der allgemeinen Formel (I).

Vorzugsweise bedeuten R¹, R², R³ und R⁴ unabhängig voneinander Methyl, Ethyl, Phenyl oder Phenyl, das mit 1 oder 2 Substituenten aus der Gruppe Methyl, Ethyl, Methoxy, Ethoxy, CN, F, Cl, S-Methyl, Phenyl oder Benzyl substituiert ist.

Von besonderern Interesse sind symmetrische Diketopyrrolopyrrole der Formel (I), worin R¹ und R⁴ gleich sind, und R² und R³ gleich sind, insbesondere Diketopyrrolopyrrole der Formel (I), worin R¹ und R⁴ jeweils eine Methyl- oder Ethylgruppe, und R² und R³ gleich sind und jeweils einen Phenylrest, der unsubstituiert oder durch 1 oder 2 Substituenten aus der Gruppe Methyl, Ethyl, Methoxy, Ethoxy, F, Cl, NO₂, CF₃, Phenyl oder Benzyl substituiert ist, darstellen.

Die erfindungsgemäßen Diketopyrrolopyrrolpigmente und die erfindungsgemäßen Gemische lassen sich durch Umsetzung eines Bernsteinsäurediesters mit einem Nitril der Formel (II) oder (III), 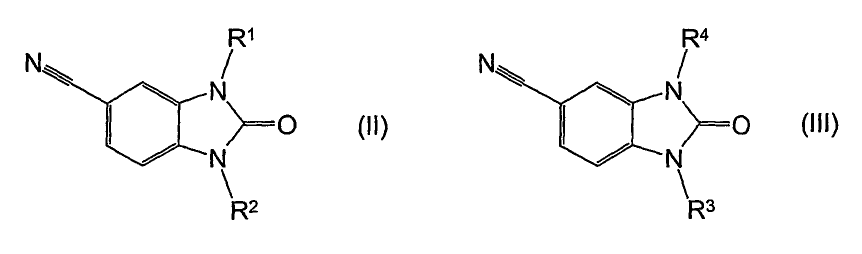 oder mit einem Gemisch von 2, 3 oder 4 verschiedenen Nitrilen der Formel (II) oder (III), in einem organischen Lösemittel in Gegenwart einer starken Base und anschließender Hydrolyse herstellen, wobei R¹ bis R⁴ die obengenannten Bedeutungen haben.

Bei den zu verwendenden Bernsteinsäurediestern kann es sich um Dialkyl-, Diaryl- oder Monoalkylmonoarylester handeln, wobei auch die Bernsteinsäuredialkylester und -diarylester unsymmetrisch sein können. Bevorzugt werden symmetrische Bernsteinsäurediester, insbesondere symmetrische Bernsteinsäuredialkylester, verwendet. Liegt ein Bernsteinsäurediarylester oder -monoaryl-monoalkylester vor, so bedeutet Aryl insbesondere unsubstituiertes oder durch 1, 2 oder 3 Substituenten aus der Gruppe Halogen, wie Chlor, C₁-C₆-Alkyl, wie Methyl, Ethyl, Isopropyl, tert.-Butyl oder tert.-Amyl, oder C₁-C₆-Alkoxy, wie Methoxy oder Ethoxy, substituiertes Phenyl. Aryl bedeutet bevorzugt unsubstituiertes Phenyl. Handelt es sich um einen Bernsteinsäuredialkylester oder -monoalkyl-monoarylester, so kann Alkyl unverzweigt, verzweigt oder cyclisch sein, bevorzugt verzweigt, und vorzugsweise 1 bis 18, insbesondere 1 bis 12, vor allem 1 bis 8 und besonders bevorzugt 1 bis 5C-Atome enthalten. Alkyl ist bevorzugt sek.- oder tert.-Alkyl, wie z.B. Isopropyl, sek.-Butyl, tert.-Butyl, tert.-Amyl, Cyclohexyl, Heptyl, 2,2-Dimethylhexyl, Octyl, Decyl, Dodecyl, Tetradecyl oder Octadecyl. Beispiele für Bernsteinsäurediester sind Bernsteinsäure-dimethylester, -diethylester, -dipropylester, -dibutylester, -dipentylester, -dihexylester, -diheptylester, -dioctylester, -diisopropylester, -di-sec.-butylester, -di-tert.-butylester, -di-tert.-amylester, -di-[1,1-dimethylbutyl]-ester, -di-[1,1,3,3-tetramethylbutyl]-ester, -di-[1,1-dimethylpentyl]-ester, -di-[1-methyl-1-ethyl-butyl]-ester, -di-[1,1-diethylpropyl]-ester, -diphenylester, -di-[4-methylphenyl]-ester, -di-[2-methylphenyl]-ester, -di-[4-chlorphenyl]-ester, -di-[2,4-dichlorphenyl]-ester, -monoethylmonophenylester, -dicyclohexylester.
Insbesondere werden symmetrische Bernsteinsäuredialkylester verwendet, worin Alkyl verzweigt ist und 3 bis 5 C-Atome enthält.

Die Bernsteinsäurediester und die Nitrile sind bekannte Verbindungen und können nach bekannten Verfahren hergestellt werden.

Die Menge des eingesetzten Nitrils bzw. die Gesamtmenge der eingesetzten Nitrile sollte stöchiometrisch, bezogen auf die Menge an Bernsteinsäurediester, sein, das sind 2 Mol Nitril auf 1 Mol Bernsteinsäurediester. Es hat sich jedoch als günstig erwiesen, einen Überschuss an Nitril oder an Bernsteinsäurediester einzusetzen, um höhere Ausbeuten zu erzielen. Zweckmäßigerweise werden, bezogen auf die stöchiometrischen Mengen, bis zu 10-fache Überschüsse an Nitril oder an Bernsteinsäurediester eingesetzt, bevorzugt bis zu 5-fache Überschüsse, insbesondere bis zu 3-fache Überschüsse. Überschüssiges Nitril kann zurückgewonnen werden.

Die Umsetzung des Bernsteinsäurediesters mit dem Nitril wird in einem organischen Lösemittel durchgeführt. Als Lösemittel eignen sich beispielsweise primäre, sekundäre oder tertiäre Alkohole mit 1 bis 10 C-Atomen, wie Methanol, Ethanol,
n-Propanol, Isopropanol, n-Butanol, sek.-Butanol, tert.-Butanol, Pentanole, wie n-Pentanol oder 2-Methyl-2-butanol, Hexanole, wie 2-Methyl-2-pentanol oder 3-Methyl-3-pentanol, 2-Methyl-2-hexanol, 3-Ethyl-3-pentanol, Octanole, wie 2,4,4-Trimethyl-2-pentanol, Cyclohexanol, oder Glykole, wie Ethylenglykol, Diethylenglykol, Propylenglykol, Dipropylenglykol oder Glycerin, oder Polyglykole, wie Polyethylenglykole oder Polypropylenglykole, Ether, wie Methylisobutylether, Tetrahydrofuran, Dimethoxyethan oder Dioxan, Glykolether, wie Monomethyl- oder Monoethylether des Ethylen- oder Propylenglykols, Diethylenglykolmonomethylether oder Diethylenglykol-monoethylether, Butylglykole oder Methoxybutanol, dipolar-aprotische Lösemittel, beispielsweise Säureamide wie Dimethylformamid, N,N-Dimethylacetamid oder N-Methylpyrrolidon, Harnstoffderivate wie Tetramethylharnstoff, aliphatische oder aromatische Kohlenwasserstoffe, wie Cyclohexan oder Benzol oder durch Alkyl, Alkoxy, Nitro oder Halogen substituiertes Benzol, wie Toluol, Xylole, Ethylbenzol, Anisol, Nitrobenzol, Chlorbenzol, o-Dichlorbenzol oder 1,2,4-Trichlorbenzol, aromatische N-Heterocyclen, wie Pyridin, Picolin oder Chinolin, sowie Hexamethylphosphorsäuretriamid, 1,3-Dimethyl-2-imidazolidinon, Dimethylsulfoxid oder Sulfolan.
Zweckmäßigerweise werden 2 bis 25 Gew.-Teile Lösemittel auf 1 Gew.-Teil der Reaktionsteilnehmer verwendet. Die Menge sollte ausreichen, um eine rührbare Suspension zu gewährleisten. Zudem ist es auch möglich, das umzusetzende Nitril der Formel (II) bzw. (III) gleichzeitig als Lösemittel zu verwenden, falls es in dem Temperaturbereich flüssig ist, in dem die Umsetzung erfolgt.
Im erfindungsgemäßen Verfahren wird bevorzugt ein Alkohol als Lösemittel verwendet, insbesondere ein sekundärer oder tertiärer Alkohol. Bevorzugte tertiäre Alkohole sind tert.-Butanol und tert.-Amylalkohol.
Es können auch Gemische der Lösemittel eingesetzt werden, insbesondere der bevorzugten sekundären und tertiären Alkohole mit aromatischen Kohlenwasserstoffen, wie Toluol oder Xylol, oder mit durch Halogen substituiertem Benzol, wie Chlorbenzol.
Das erfindungsgemäße Verfahren wird in Gegenwart einer starken Base durchgeführt. Geeignete starke Basen sind insbesondere die Alkalimetalle selbst, wie Lithium, Natrium oder Kalium, oder Alkaliamide, wie Lithium-, Natrium- oder Kaliumamid, oder Alkalihydride, wie Lithium-, Natrium- oder Kaliumhydrid, oder Erdalkali- oder Alkalialkoholate, die sich insbesondere von primären, sekundären oder tertiären aliphatischen Alkoholen mit 1 bis 10°C-Atomen ableiten, wie z.B. Lithium-, Natrium- oder Kaliummethylat, -ethylat, -n-propylat, -isopropylat, n-butylat, -sek.-butylat, -tert.-butylat, -2-methyl-2-butylat, -2-methyl-2-pentylat, -3-methyl-3-pentylat, -3-ethyl-3-pentylat. Auch Gemische der genannten Basen können verwendet werden.
Im erfindungsgemäßen Verfahren werden als starke Base bevorzugt Alkalialkoholate verwendet, wobei Alkali insbesondere Natrium oder Kalium bedeutet, und das Alkoholat sich bevorzugt von einem sekundären oder tertiären Alkohol ableitet. Besonders bevorzugte starke Basen sind daher z.B. Natrium- oder Kalium-isopropylat, -sek.-butylat, -tert.-butylat und -tert.-amylat. Dabei können die Alkalialkoholate auch in situ hergestellt werden, indem der entsprechende Alkohol mit dem Alkalimetall, Alkalihydrid oder Alkaliamid umsetzt wird.
Im erfindungsgemäßen Verfahren kann die starke Base in einer Menge von 0,1 bis 10 Mol, bevorzugt 1,9 bis 5,0 Mol, bezogen auf 1 Mol des im Unterschuss eingesetzten Reaktionspartners, eingesetzt werden. Obwohl grundsätzlich stöchiometrische Mengen an Base genügen, beeinflusst in vielen Fällen ein Basenüberschuss die Ausbeute günstig.

Die erfindungsgemäße Umsetzung des Bersteinsäurediesters mit den Nitrilen der Formel (II) oder der Formel (III) wird insbesondere bei einer Temperatur von 60 bis 200°C, vorzugsweise 80 bis 140°C, durchgeführt, gegebenenfalls unter Druck. Zur Umsetzung können die einzelnen Komponenten in beliebiger Reihenfolge zugegeben werden, vorzugsweise bei Reaktionstemperatur. Es ist auch möglich, bei tieferer Temperatur alle Komponenten vorzulegen und dann das Gemisch in den Bereich der Reaktionstemperatur zu erwärmen. Eine bevorzugte Ausführungsform, die sich in der Regel besonders günstig auf die Ausbeute auswirkt, besteht darin, dass das umzusetzende Nitril zusammen mit der Base vorgelegt wird und der Bernsteinsäurediester im Bereich der Reaktionstemperatur zudosiert wird. Eine weitere Möglichkeit besteht darin, dass der Bernsteinsäurediester und das umzusetzende Nitril gleichzeitig zur vorgelegten Base bei Reaktionstemperaturzudosiert werden. Es ist möglich, das erfindungsgemäße Verfahren batchweise oder kontinuierlich durchzuführen. Insbesondere bei Bernsteinsäurediestern mit niederen Alkylresten und bei Alkoholaten, die sich aus niederen Alkoholen ableiten, wie z.B. Methanol, Ethanol, n-Propanol, Isopropanol oder tert.-Butanol, kann es sich als notwendig erweisen, den bei der Umsetzung entstehenden niederen Alkohol laufend aus dem Reaktionsmilieu zu entfernen, um höhere Ausbeuten zu erzielen.
Wird als Lösemittel ein Alkohol und als Base ein Alkoholat verwendet, so kann es vorteilhaft sein, einen Alkohol und ein Alkoholat mit gleichen Alkylteilen zu wählen. Ebenso vorteilhaft kann es sein, wenn zudem noch der Bernsteinsäurediester ebensolche Alkylgruppen enthält.

Zur Hydrolyse des Umsetzungsproduktes können als Hydrolysemittel Wasser, ein oder mehrere organische, protische Lösemittel, oder ein oder mehrere Säuren verwendet werden. Als protische Lösemittel kommen z.B. Alkohole, vorzugsweise mit 1 bis 4 C-Atomen, wie Methanol oder Ethanol, in Betracht. Als Säuren kommen organische Säuren, wie z.B. aliphatische oder aromatische Carbon- oder Sulfonsäuren, in Betracht, wie beispielsweise Ameisensäure, Essigsäure, Propionsäure, Buttersäure, Hexansäure, Oxalsäure, Citronensäure, Benzoesäure, Phenylessigsäure, Benzolsulfonsäure oder p-Toluolsulfonsäure, sowie anorganische Säuren, wie z.B. Salzsäure, Schwefelsäure oder Phosphorsäure. Vorzugsweise wird zur Hydrolyse eine organische Säure, insbesondere eine aliphatische Carbonsäure, wie Essigsäure und Ameisensäure, benutzt. Es können auch Wasser, organisches protisches Lösemittel und/oder Säure in beliebigen Kombinationen verwendet werden. Die Hydrolyse kann auch in Gegenwart von organischen aprotischen Lösemitteln durchgeführt werden.
Die Hydrolyse kann direkt durch Zugabe eines Hydrolysemittels zur Reaktionssuspension, oder indirekt, durch Zugabe der Reaktionssuspension zum Hydrolysemittel, geschehen. Die Hydrolysemittel Wasser, organisches, protisches Lösemittel und Säure können in beliebiger Reihenfolge und auch als Mischungen zugegeben und/oder vorgelegt werden. Auch eine gleichzeitige Zugabe einzelner Komponenten zu einer Vorlage ist möglich, beispielsweise können Säure und die Reaktionssuspension gleichzeitigzum vorgelegten Wasser und/oder Alkohol gegeben werden.
Es kann von Vorteil sein, einen Puffer während der Hydrolyse zu verwenden, wie beispielsweise einen Phosphat-, Acetat-, Citronensäure- oder Triethanolamin-Puffer.
Die Temperatur bei der Hydrolyse kann -20°C bis 200°C betragen, bevorzugt -5 bis 180°C, insbesondere 0 bis 160°C, gegebenenfalls findet die Hydrolyse unter Druck statt. Dabei können Reaktionssuspension und Hydrolysemittel auch unterschiedliche Temperatur haben. Beispielsweise kann die Hydrolyse auch mittels Wasserdampf geschehen.
Die Gesamtmenge an Hydrolysemittel beträgt zweckmäßigerweise eine mindestens stöchiometrische Menge bezogen auf Base. Beispielsweise kann Wasser und/oder ein organisches, protisches Lösemittel zwischen 0,5 und 50 Gew.-Teilen auf 1 Teil des entstandenen Pigments eingesetzt werden. Die Säure wird zweckmäßigerweise in 0,1 bis 10-fachem molarem Überschuss, bezogen auf Base, eingesetzt. Liegt nach der Hydrolyse eine wasserhaltige Suspension vor, kann der pH sowohl im alkalischen, neutralen als auch im sauren Bereich liegen.

Je nach Verfahrensweise fällt bei der Hydrolyse das Diketopyrrolopyrrol der Formel (I) als Pigment, feinteiliges Präpigment oder grobkristallines Rohpigment aus. Nach der Hydrolyse vorliegende Pigmente können in üblicher Weise durch Filtration isoliert werden. Vor der Isolierung des Pigments kann das Lösemittel destillativ, gegebenenfalls unter reduziertem Druck, oder auch durch Wasserdampfdestillation entfernt werden. Dies kann bereits während der Hydrolyse geschehen.

Präpigmente und Rohpigmente müssen noch einer Nachbehandlung unterzogen werden. Dazu kann die Hydrolysesuspension direkt eingesetzt werden, das Pigment kann aber auch zuerst isoliert und dann nachbehandelt werden.
Die Nachbehandlung kann eine thermische Nachbehandlung in Wasser und/oder organischem Lösemittel bei beliebigem pH und bei einer Temperatur von 50 bis 250°C, vorzugsweise von 80 bis 190°C, gegebenenfalls unter Druck, für 10 Minuten bis 48 Stunden, vorzugsweise für 30 Minuten bis 8 Stunden, oder eine Mahlung, oder eine Kombination dieser beiden Prozesse sein.
Die Mahlung kann sowohl durch Trocken- als auch durch Nassmahlung erfolgen. Für die Trockenmahlung eignen sich alle diskontinuierlichen und kontinuierlichen Schwing- oder Rollmühlen und für die Nassmahlung alle diskontinuierlichen und kontinuierlichen Rührwerkslcugel-, Roll- und Schwingmühlen sowie Kneter. Die Nassmahlung findet in Wasser und/oder organischem Lösemittel bei beliebigem pH statt.
Bevorzugt wird eine Nassmahlung mit hohem Energieeintrag durchgeführt, z.B. auf einer Rührwerkskugelmühle mit einer Leistungsdichte von über 1,0 kW pro Liter Mahlraum und einer Rührwerksumfangsgeschwindigkeit von über 12 m/s.
Die Zugabe des Hydrolysemittels oder der Reaktionssuspension kann auch portionsweise und sequentiell erfolgen, so dass zwischen den einzelnen Portionen eine Zwischenbehandlung, wie beispielsweise ein längeres Rühren, gegebenenfalls unter erhöhter Temperatur, erfolgen kann.
Vorzugsweise wird zur Nachbehandlung die Hydrolysesuspension ohne Zwischenisolierung des Diketopyrrolopyrrols verwendet.

Zur Verbesserung der coloristischen Eigenschaften und zur Erzielung bestimmter anwendungstechnischer Effekte können Hilfsmittel wie beispielsweise Tenside, pigmentäre und nichtpigmentäre Dispergiermittel, Füllstoffe, Stellmittel, Harze, Wachse, Entschäumer, Antistaubmittel, Extender, Farbmittel zum Nuancieren, Konservierungsmittel, Trocknungsverzögerungsmittel, Additive zur Steuerung der Rheologie, Netzmittel, Antioxidantien, UV-Absorber, Lichtstabilisatoren, oder eine Kombination davon eingesetzt werden.
Die Zugabe von Hilfsmitteln kann zu einem beliebigen Zeitpunkt vor, während oder nach der Umsetzung, Hydrolyse und/oder Nachbehandlung erfolgen, auf einmal oder in mehreren Portionen.
Die Gesamtmenge der zugegebenen Hilfsmittel kann 0 bis 40 Gew.-%, bevorzugt 1 bis 30 Gew.-%, besonders bevorzugt 2,5 bis 25 Gew.-%, bezogen auf das Diketopyrrolopyrrolpigment, betragen.

Als Tenside kommen anionische oder anionaktive, kationische oder kationaktive und nichtionische Substanzen oder Mischungen dieser Mittel in Betracht. Bevorzugt sind solche Tenside oder Mischungen von Tensiden, die nicht schäumen.
Als anionaktive Substanzen kommen beispielsweise Fettsäuretauride, FettsäureN-methyltauride, Fettsäureisethionate, Alkylphenylsulfonate, Alkylnaphthalinsulfonate, Alkylphenolpolyglykolethersulfate, Fettalkoholpolyglykotethersulfate, Fettsäureamid-polyglykolethersulfate, Alkylsulfosuccinamate, Alkenylbernsteinsäurehalbester, Fettalkoholpolyglykolethersulfosuccinate, Alkansulfonate, Fettsäureglutamate, Alkylsulfosuccinate, Fettsäuresarkoside; Fettsäuren, beispielsweise Palmitin-, Stearin- und Ölsäure; Seifen, beispielsweise Alkalisalze von Fettsäuren, Naphthensäuren und Harzsäuren, beispielsweise Abietinsäure, alkalilösliche Harze, beispielsweise kolophoniummodifizierte Maleinatharze und Kondensationsprodukte auf Basis von Cyanurchlorid, Taurin, N,N'-Diethylaminopropylamin und p-Phenylendiamin in Betracht. Besonders bevorzugt sind Harzseifen, d.h. Alkalisalze von Harzsäuren.
Als kationaktive Substanzen kommen beispielsweise quaternäre Ammoniumsalze, Fettaminoxalkylate, oxalkylierte Polyamine, Fettaminpolyglykolether, Fettamine, von Fettaminen oder Fettalkoholen abgeleitete Di- und Polyamine und deren Oxalkylate, von Fettsäuren abgeleitete Imidazoline, und Salze dieser kationenaktiven Substanzen, wie beispielsweise Acetate, in Betracht.
Als nichtionogene Substanzen kommen beispielsweise Aminoxide, Fettalkoholpolyglykolether, Fettsäurepolyglykolester, Betaine, wie Fettsäureamid-N-propyl-betaine, Phosphorsäureester von aliphatischen und aromatischen Alkoholen, Fettalkoholen oder Fettalkoholpolyglykolethern, Fettsäureamidethoxylate, Fettalkohol-alkylenoxid-Addukte und Alkylphenolpolyglykolether in Betracht.

Mit nichtpigmentären Dispergiermitteln sind Substanzen gemeint, die strukturell nicht durch chemische Modifikation von organischen Pigmenten abgeleitet sind. Sie werden als Dispergiermittel entweder bereits bei der Herstellung von Pigmenten, oft aber auch bei der Einarbeitung der Pigmente in die zu färbenden Anwendungsmedien, beispielsweise bei der Herstellung von Lacken oder Druckfarben durch Dispergierung der Pigmente in den entsprechenden Bindemitteln, zugegeben. Es können polymere Substanzen, beispielsweise Polyolefine, Polyester, Polyether, Polyamide, Polyimine, Polyacrylate, Polyisocyanate, Block-Copolymere daraus, Copolymere aus den entsprechenden Monomeren oder Polymere einer Klasse sein, die mit wenigen Monomeren einer anderen Klasse modifiziert sind. Diese polymeren Substanzen tragen polare Ankergruppen wie beispielsweise Hydroxy-, Amino-, Imino- und Ammoniumgruppen, Carbonsäure- und Carboxylatgruppen, Sulfonsäure- und Sulfonatgruppen oder Phosphonsäure- und Phosphonatgruppen, und können auch mit aromatischen, nicht pigmentären Substanzen modifiziert sein. Nichtpigmentäre Dispergiermittel können des weiteren auch chemisch mit funktionellen Gruppen modifizierte aromatische, nicht von organischen Pigmenten abgeleitete Substanzen sein. Derartige nichtpigmentäre Dispergiermittel sind dem Fachmann bekannt und zum Teil im Handel erhältlich (z.B. Solsperse^{®}, Avecia; Disperbyk^{®}, Byk, Efka^{®}, Efka). Es sollen im Folgenden stellvertretend einige Typen genannt werden, zum Einsatz können jedoch prinzipiell beliebige andere, beschriebene Substanzen kommen, beispielsweise Kondensationsprodukte aus Isocyanaten und Alkoholen, Di- oder Polyolen, Aminoalkoholen oder Di- oder Polyaminen, Polymere aus Hydroxycarbonsäuren, Copolymere aus Olefinmonomeren oder Vinylmonomeren und ethylenisch ungesättigten Carbonsäuren Estern, urethanhaltige Polymere von ethylenisch ungesättigten Monomeren, urethanmodifizierte Polyester, Kondensationsprodukte auf Basis von Cyanurhalogeniden, Nitroxylverbindungen enthaltende Polymere, Polyesteramide, modifizierte Polyamide, modifizierte Acrylpolymere, Kammdispergiermittel aus Polyestern und Acrylpolymeren, Phosphorsäureester, von Triazin abgeleitete Polymere, modifizierte Polyether, oder von aromatischen, nichtpigmentären Substanzen abgeleitete Dispergiermittel. Dabei werden diese Grundstrukturen vielfach weiter modifiziert, beispielsweise durch chemische Umsetzung mit weiteren, funktionelle Gruppen tragenden Substanzen oder durch Salzbildung.

Mit pigmentären Dispergiermitteln sind Pigmentdispergatoren gemeint, die sich von einem organischen Pigment als Grundkörper ableiten und durch chemische Modifizierung dieses Grundkörpers hergestellt werden, beispielsweise saccharinhaltige Pigmentdispergatoren, piperidylhaltige Pigmentdispergatoren, von Naphthalin oder Perylen abgeleitete Pigmentdispergatoren, Pigmentdispergatoren mit funktionellen Gruppen, die über eine Methylengruppe mit dem Pigmentgrundkörper verknüpft sind, mit Polymeren chemisch modifizierte Pigmentgrundkörper, Sulfosäure-, Sulfonamid- oder Sulfosäureestergruppenhaltige Pigmentdispergatoren, Ether- oder Thioethergruppen-haltige Pigmentdispergatoren, oder Carbonsäure-, Carbonsäureester- oder Carbonamidgruppen-haltige Pigmentdispergatoren.

Gemische von Verbindungen der Formel (I) können auch durch gemeinsame Hydrolyse von unabhängig voneinander hergestellten, verschiedenen Reaktionslösungen hergestellt werden, mit oder ohne vorherige Mischung der Reaktionslösungen, oder auch durch gemeinsames Umfällen von zwei oder mehr Verbindungen der Formel (I).

Unsymmetrisch substituierte Diketopyrrolopyrrole der Formel (I) können auch hergestellt werden, in dem an Stelle der Umsetzung von Nitril mit Bernsteinsäurediester ein Ester der Formel (IV) oder der Formel (V), 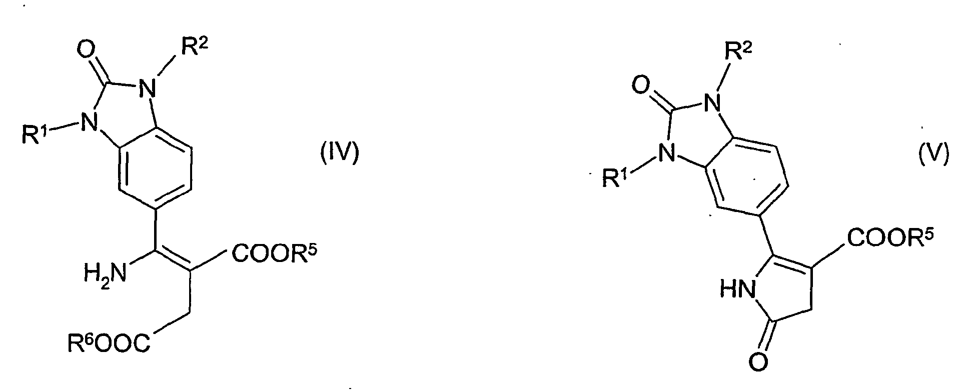 mit einem Nitril der Formel (III) in einem organischen Lösemittel in Gegenwart einer starken Base und anschließender Hydrolyse umgesetzt wird, wobei R¹ und R² die angegebene Bedeutung haben und R⁵ und R⁶ einen gegebenenfalls substituierten Alkyl- oder Arylrest, vorzugsweise die bei den Bernsteinsäurediestern genannten Esterreste, bedeuten.

Dabei kann die Umsetzung analog den für die Umsetzung von Bernsteinsäurediestern mit Nitril beschriebenen Bedingungen durchgeführt werden.

Die Ester der Formel (IV) und der Formel (V) können analog den in US-B1-6,207,697 oder WO 00/34248 offenbarten Verfahren hergestellt werden.

Die erfindungsgemäßen Diketopyrrolopyrrolpigmente können als vorzugsweise wässrige Presskuchen zum Einsatz kommen, in der Regel handelt es sich jedoch um feste Systeme von rieselfähiger, pulverförmiger Beschaffenheit oder um Granulate.

Die erfindungsgemäßen Diketopyrrolopyrrolpigmente zeichnen sich besonders in Lacken durch hervorragende coloristische und rheologische Eigenschaften aus, insbesondere durch eine hervorragende Rheologie, hohe Transparenz, gutes Glanzverhalten, hohe Farbstärke, einwandfreie Überlackierechtheiten sowie sehr gute Licht- und Wetterechtheit. Sie können in lösemittelhaltigen und in wässrigen Systemen eingesetzt werden. Auch in Kunststoffen und Drucksystemen zeigen sie gute Eigenschaften und sind somit universell einsetzbar.

Die erfindungsgemäß hergestellten Diketopyrrolopyrrolpigmente lassen sich zum Pigmentieren von hochmolekularen organischen Materialien natürlicher oder synthetischer Herkunft einsetzen, beispielsweise von Kunststoffen, Harzen, Lacken, Anstrichfarben oder elektrophotographischen Tonern und Entwicklern, sowie von Tinten und Druckfarben.

Hochmolekulare organische Materialien, die mit den genannten Pigmenten pigmentiert werden können, sind beispielsweise Celluloseether und -ester, wie Ethylcellulose, Nitrocellulose, Celluloseacetat oder Cellulosebutyrat, natürliche Harze oder Kunstharze, wie Polymerisationsharze oder Kondensationsharze, beispielsweise Aminoplaste, insbesondere Harnstoff-und Melaminformaldehydharze, Alkydharze, Acrylharze, Phenoplaste, Polycarbonate, Polyolefine, wie Polystyrol, Polyvinylchlorid, Polyethylen, Polypropylen, Polyacrylnitril, Polyacrylsäureester, Polyamide, Polyurethane oder Polyester, Gummi, Casein, Silikon und Silikonharze, einzeln oder in Mischungen. Dabei spielt es keine Rolle, ob die erwähnten hochmolekularen organischen Verbindungen als plastische Massen, Schmelzen oder in Form von Spinnlösungen, Lacken, Anstrichstoffen oder Druckfarben vorliegen. Je nach Verwendungszweck erweist es sich als vorteilhaft, die erfindungsgemäß erhaltenen Pigmente als Blend oder in Form von Präparationen oder Dispersionen zu benutzen. Bezogen auf das zu pigmentierende, hochmolekulare organische Material setzt man die erfindungsgemäßen Pigmente in einer Menge von 0,05 bis 30 Gew.-%, vorzugsweise 0,1 bis 15 Gew.-% ein.
Die erfindungsgemäßen Pigmente sind auch geeignet als Farbmittel in elektrophotographischen Tonern und Entwicklern, wie beispielsweise Ein- oder Zweikomponentenpulvertonern (auch Ein- oder Zweikomponenten-Entwickler genannt), Magnettoner, Flüssigtoner, Polymerisationstoner sowie Spezialtoner. Typische Tonerbindemittel sind Polymerisations-, Polyadditions- und Polykondensationsharze, wie Styrol-, Styrolacrylat-, Styrolbutadien-, Acrylat-, Polyester-, Phenol-Epoxidharze, Polysulfone, Polyurethane, einzeln oder in Kombination, sowie Polyethylen und Polypropylen, die noch weitere Inhaltsstoffe, wie Ladungssteuermittel, Wachse oder Fließhilfsmittel, enthalten können oder im Nachhinein mit diesen Zusätzen modifiziert werden.

Des weiteren sind die erfindungsgemäßen Pigmente geeignet als Farbmittel in Pulver und Pulverlacken, insbesondere in triboelektrisch oder elektrokinetisch versprühbaren Pulverlacken, die zur Oberflächenbeschichtung von Gegenständen aus beispielsweise Metall, Holz, Kunststoff, Glas, Keramik, Beton, Textilmaterial, Papier oder Kautschuk zur Anwendung kommen.
Als Pulverlackharze werden typischerweise Epoxidharze, carboxyl- und hydroxylgruppenhaltige Polyesterharze, Polyurethan- und Acrylharze zusammen mit üblichen Härtern eingesetzt. Auch Kombinationen von Harzen finden Verwendung. So werden beispielsweise häufig Epoxidharze in Kombination mit carboxyl- und hydroxylgruppenhaltigen Polyesterharzen eingesetzt. Typische Härterkomponenten (in Abhängigkeit vom Harsystem) sind beispielsweise Säureanhydride, Imidazole sowie Dicyandiamid und deren Abkömmlinge, verkappte Isocyanate, Bisacylurethane, Phenol- und Melaminhare, Triglycidylisocyanurate, Oxazoline und Dicarbonsäuren.

Außerdem sind die erfindungsgemäßen Pigmente als Farbmittel in Ink-Jet Tinten auf wässriger und nichtwässriger Basis sowie in solchen Tinten, die nach dem Hot-melt-Verfahren arbeiten, geeignet. Ink-Jet-Tinten enthalten im allgemeinen insgesamt 0,5 bis 15 Gew.-%, vorzugsweise 1,5 bis 8 Gew.-%, (trocken gerechnet) einer oder mehrerer der erfindungsgemäß hergestellten Verbindungen.
Mikroemulsionstinten basieren auf organischen Lösemitteln, Wasser und ggf. einer zusätzlichen hydrotropen Substanz (Grenzflächenvermittler). Mikroemulsionstinten enthalten im allgemeinen 0,5 bis 15 Gew.-%, vorzugsweise 1,5 bis 8 Gew.-%, einer oder mehrerer der erfindungsgemäß hergestellten Verbindungen, 5 bis 99 Gew.-% Wasser und 0,5 bis 94,5 Gew.-% organisches Lösungsmittel und/oder hydrotrope Verbindung.
"Solvent based" Ink-Jet-Tinten enthalten vorzugsweise 0,5 bis 15 Gew.-% einer oder mehrerer erfindungsgemäß hergestellter Verbindungen, 85 bis 99,5 Gew.-% organisches Lösungsmittel und/oder hydrotrope Verbindungen.
Hot-Melt-Tinten basieren meist auf Wachsen, Fettsäuren, Fettalkoholen oder Sulfonamiden, die bei Raumtemperatur fest sind und bei Erwärmen flüssig werden, wobei der bevorzugte Schmelzbereich zwischen ca. 60°C und ca. 140°C liegt. Hot-Melt ink-Jet-Tinten bestehen z.B. im wesentlichen aus 20 bis 90 Gew.-% Wachs und 1 bis 10 Gew.-% einer oder mehrerer der erfindungsgemäß hergestellten Verbindungen. Weiterhin können 0 bis 20 Gew.-% eines zusätzlichen Polymers (als "Farbstofflöser"), 0 bis 5 Gew.-% Dispergierhilfsmittel, 0 bis 20 Gew.-% Viskositätsveränderer, 0 bis 20 Gew.-% Plastifizierer, 0 bis 10 Gew.-% Klebrigkeitszusatz, 0 bis 10 Gew.-% Transparenzstabilisator (verhindert z.B. Kristallisation der Wachse) sowie 0 bis 2 Gew.-% Antioxidans enthalten sein. Typische Zusatzstoffe und Hilfsmittel sind z.B. in US-PS 5,560,760 beschrieben.

Weiterhin sind die erfindungsgemäßen Pigmente auch als Farbmittel für Farbfilter sowohl für die additive wie subtraktive Farberzeugung sowie für elektronische Tinten ("electronic inks") geeignet.

Zur Beurteilung der Eigenschaften der Pigmente auf dem Kunststoffgebiet wurde aus der Vielzahl der bekannten Kunststoffe Weichpolyvinylchlorid (PVC) ausgewählt.
Zur Beurteilung der Eigenschaften der Pigmente auf dem Drucksektor wurde aus der Vielzahl der bekannten Drucksysteme ein Tiefdrucksystem auf Nitrocellulosebasis (NC) ausgewählt.

Zur Beurteilung der Eigenschaften der Pigmente auf dem Lacksektor wurden aus der Vielzahl der bekannten Lacke ein High-Solid-Acrylharzeinbrennlack auf Basis einer nichtwässrigen Dispersion (HS), ein wässriger Lack auf Polyurethanbasis (PUR), sowie ein Alkyd-Melaminharz-Lack (AM) auf Basis eines mittelöligen Alkydharzes und eines butanolveretherten Melaminharzes ausgewählt.

Die Bestimmung der Farbstärke und des Farbtons erfolgte nach DIN 55986.
Die Rheologie des Mahlguts nach der Dispergierung (millbase-Rheologie) wurde visuell anhand der folgenden fünfstufigen Skala bewertet.
- 5: dünnflüssig
- 4: flüssig
- 3: dickflüssig
- 2.: leicht gestockt
- 1: gestockt

Die Bestimmung der Überlackierechtheit erfolgte nach DIN 53221.

Die Bestimmung der Viskosität erfolgte nach dem Verdünnen des Mahlguts auf die Pigmentendkonzentration mit dem Viskospatel nach Rossmann, Typ 301 der Firma Erichsen.

In den nachfolgeneden Beispielen bedeuten Teile jeweils Gewichtsteile und Prozente jeweils Gewichtsprozente.

### Beispiel 1a

In einem Vierhalskolben werden 252,8 Teile tert-Amylalkohol wasserfrei vorgelegt. Nach Eintragen von 23 Teilen Natrium wird unter Rückfluss so lange gerührt, bis das Natrium umgesetzt ist. Bei 100°C werden 58,6 Teile des Nitrils der Formel (XX) 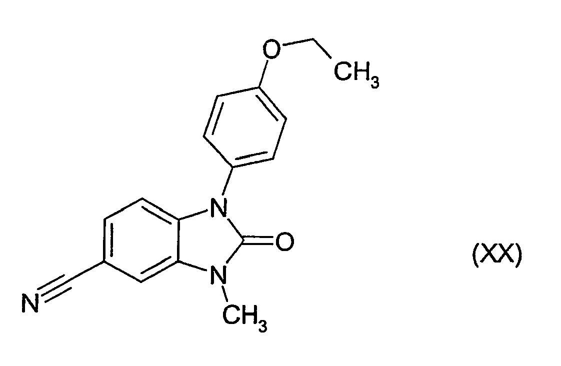 eingetragen. Innerhalb von 2 Stunden werden bei 100°C 30,4 Teile Bernsteinsäuredüsopropylester zugetropft. Dann wird 4 Stunden unter Rückfluss gerührt. Nach Abkühlen der Reaktionssuspension auf 80°C wird sie auf eine Mischung aus 300 Teilen Wasser und 400 Teilen Methanol von 25°C gegossen. Die Hydrolysesuspension wird 6,5 Stunden unter Rückfluss gerührt. Nach Abkühlen auf Raumtemperatur werden 320 Teile Methanol zugegeben und verrührt, dann wird die Suspension filtriert, das Pigment mit Methanol gewaschen. Der Presskuchen wird in 1280 Teilen Methanol suspendiert und verrührt, die Suspension wird filtriert, das Pigment mit Methanol und Wasser gewaschen und bei 80°C getrocknet.
Man erhält 36,6 Teile Diketopyrrolopyrrolpigment der Formel (XXI).

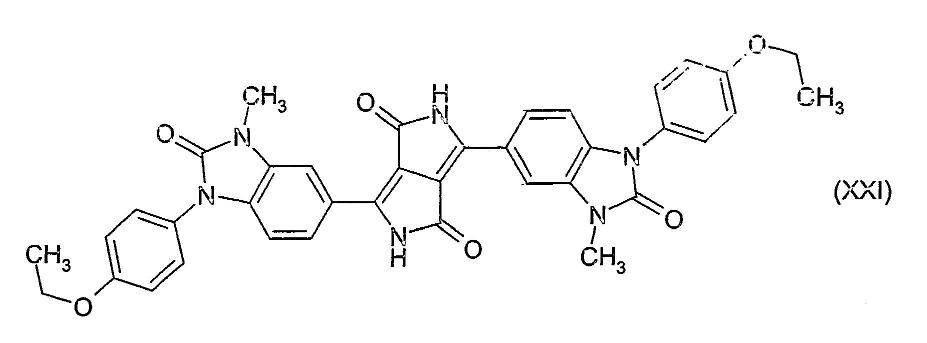

Das Pigment liefert im HS-Lack transparente und farbstarke Lackierungen mit reinem, orangem Farbton und einwandfreier Überlackierechtheit. Die Metalliclackierung ist brillant und farbstark und hat eine goldgelben Farbton. Im PUR-Lack erhält man brillante, farbstarke Metalliclackierungen mit goldgelbem Farbton, die Rheologie ist sehr gut und wird mit 5 bewertet.

### Beispiel 1b (Vergleichsbeispiel)

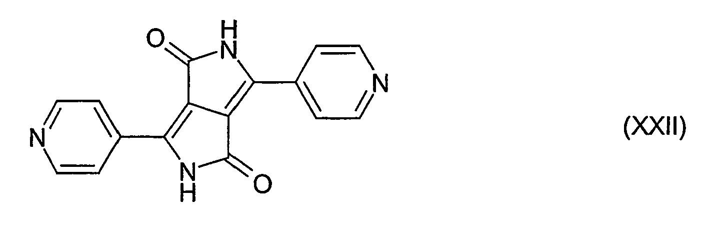

Das Diketopyrrolopyrrol der Formel (XXII) wurde gemäß EP 94911, Beispiel 12, hergestellt und gegen das Diketopyrrolopyrrol der Formel (XXI), hergestellt gemäß Beispiel 1a, verglichen. Im HS-Lack sind die Lackierungen wesentlich deckender, farbschwächer und haben einen bräunlich-roten und wesentlich trüberen Farbton. Auch die Wetterechtheit ist schlechter. Im PUR-Lack wird die Rheologie lediglich mit 2 bis 3 bewertet.

### Beispiel to (Vergleichsbeispiel)

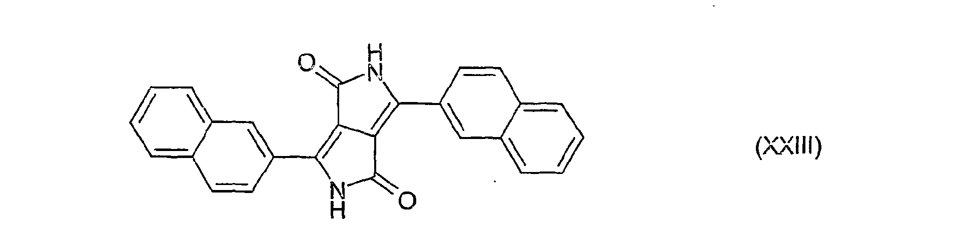

Das Diketopyrrolopyrrol der Formel (XXIII) wurde gemäß EP 94911, Beispiel 14, hergestellt und gegen das Diketopyrrolopyrrol der Formel (XXI), hergestellt gemäß Beispiel 1a, verglichen. Im HS-Lack sind die Lackierungen wesentlich deckender und haben einen bräunlich-violetten und wesentlich trüberen Farbton. Der Metallic-Lack ist farbschwach mit grau-braunem Farbton. Auch im PUR-Lack erhält man eine ähnliche, deutlich unterlegene Coloristik.

### Beispiel 2

In einem Vierhalskolben werden 189,6 Teile tert-Amylalkohol wasserfrei vorgelegt. Nach Eintragen von 17,3 Teilen Natrium wird unter Rückfluss so lange gerührt, bis das Natrium umgesetzt ist. Bei 100°C werden 41,9 Teile des Nitrils der Formel (XXIV) 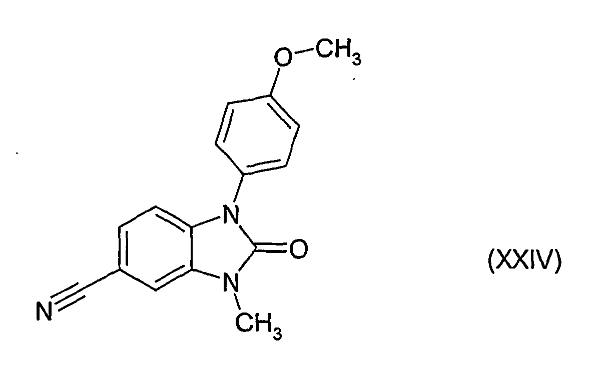 eingetragen. Innerhalb von 2 Stunden werden bei 100°C 22,8 Teile Bernsteinsäurediisopropylester zugetropft. Dann wird 4 Stunden unter Rückfluss gerührt. Nach Abkühlen der Reaktionssuspension auf 80°C wird sie auf eine Mischung aus 225 Teilen Wasser und 300 Teilen Methanol von 25°C gegossen.

Die Hydrolysesuspension wird 6,5 Stunden unter Rückfluss gerührt. Nach Abkühlen auf Raumtemperatur werden 240 Teile Methanol zugegeben und verrührt, dann wird die Suspension filtriert, das Pigment mit Methanol gewaschen. Der Presskuchen wird in 960 Teilen Methanol suspendiert und verrührt, die Suspension wird filtriert, das Pigment mit Methanol und Wasser gewaschen und bei 80°C getrocknet.
Man erhält 26,8 Teile Diketopyrrolopyrrolpigment der Formel (XXV).

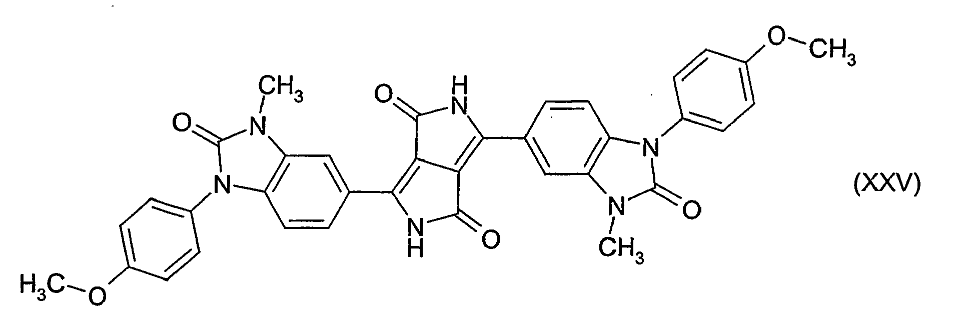

Das Pigment liefert im HS-Lack transparente Lackierungen mit reinem, orangen Farbton und einwandfreier Überlackierechtheit.

### Beispiel 3

In einem Vierhalskolben werden 189,6 Teile tert.-Amylalkohol wasserfrei vorgelegt. Nach Eintragen von 17,3 Teilen Natrium wird unter Rückfluss so lange gerührt, bis das Natrium umgesetzt ist. Bei 100°C werden 5 Teile des Nitrils der Formel (XXIV) und 38,6 Teile des Nitrils der Formel (XX) eingetragen. In 2 Stunden werden bei 100°C 22,8 Teile Bernsteinsäurediisopropylester zugetropft. Dann wird 4 Stunden unter Rückfluss gerührt. Nach Abkühlen der Reaktionssuspension auf 80°C wird sie auf eine Mischung aus 225 Teilen Wasser und 300 Teilen Methanol von 25°C gegossen. Die Hydrolysesuspension wird 2 Stunden unter Rückfluss gerührt. Nach Abkühlen auf Raumtemperatur werden 240 Teile Methanol zugegeben und verrührt, dann wird die Suspension filtriert, das Pigment mit Methanol gewaschen. Der Presskuchen wird in 960 Teilen Methanol suspendiert und verrührt, die Suspension wird filtriert, das Pigment mit Methanol und Wasser gewaschen und bei 80°C getrocknet.

Man erhält 25,9 Teile Diketopyrrolopyrrolpigment, dass aus einem Gemisch von Pigmenten der Formel (XXI), (XXV) und (XXVI) 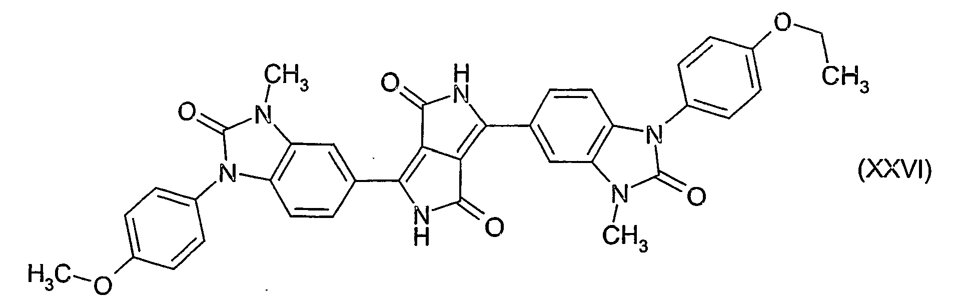 besteht. Das Pigment liefert im PUR-Lack transparente Lackierungen mit bordofarbenen Farbton und einwandfreier Überlackierechtheit. Im Gegensatz dazu zeigt ein handelsübliches Pigment P.R.255, hergestellt gemäß dem in EP 94911 offenbarten Verfahren, eine ungenügende Überlackierechtheit im PUR-Lack.

### Beispiel 4

In einem Vierhalskolben werden 176 Teile tert.-Amylalkohol wasserfrei vorgelegt. Nach Eintragen von 13,8 Teilen Natrium wird unter Rückfluss so lange gerührt, bis das Natrium umgesetzt ist. Bei 100°C werden 39,5 Teile des Nitrils der Formel (XXVII) 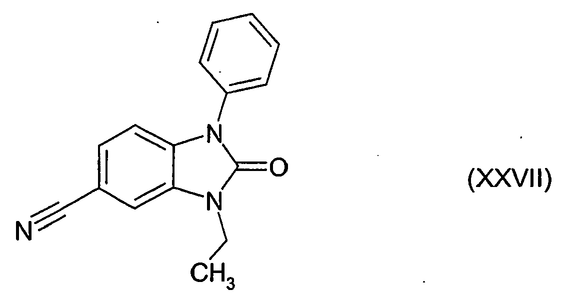 eingetragen. Innerhalb von 5 Stunden werden bei 100°C 22,7 Teile Bernsteinsäurediisopropylester zugetropft. Dann wird 1 Stunden unter Rückfluss gerührt. Nach Abkühlen der Reaktionssuspension auf 80°C wird sie auf 430 Teile Wasser von 40°C gegossen. Die Hydrolysesuspension wird 4,5 Stunden unter Rüchfluss gerührt. Nach Abkühlen auf Raumtemperatur werden 670 Teile Methanol zugegeben und verrührt, dann wird die Suspension filtriert, das Pigment mit Methanol und Wasser gewaschen und bei 80°C getrocknet.
Man erhält 32,8 Teile Giketopyrrolopyrrolpigment der Formel (XXVIII).

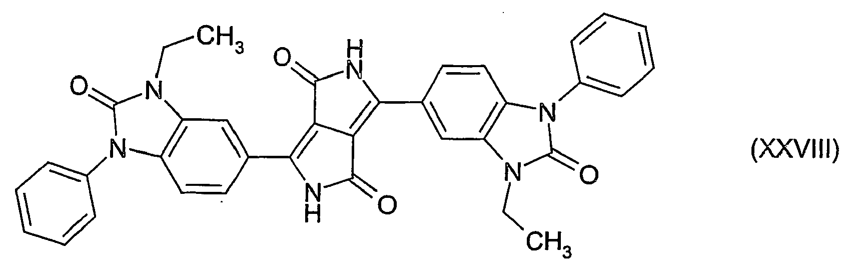

Das Pigment liefert im AM-Lack deckende und farbstarke Lackierungen mit gelbstichig-rotem Farbton und einwandfreier Überlackierechtheit, die Viskosität beträgt 3 sec. In PVC werden farbstarke Färbungen mit orangem, im NC-Druck werden farbstarke Färbungen mit gelbstichig-rotem Farbton erhalten.

## Patentansprüche

1. Diketopyrrolopyrrol der Formel (I) 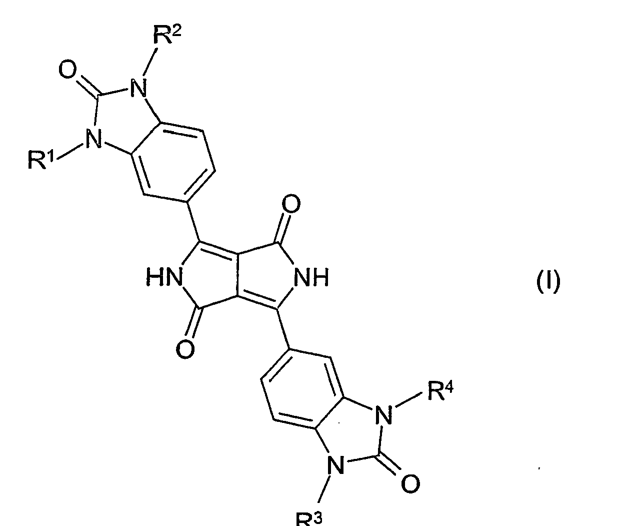 worin
R¹, R², R³ und R⁴ unabhängig voneinander einen C₁-C₄-Alkylrest oder einen substituierten oder unsubstituierten Phenylrest darstellen, wobei der Phenylrest durch 1, 2, 3 oder 4 Substituenten aus der Gruppe C₁-C₄-Alkyl, C₁-C₄-Alkoxy, CN, F, Cl, Br, NO₂, CF₃, S-C₁-C₄-Alkyl, Phenyl oder (C₁-C₂)-Alkylenphenyl substituiert sein kann,
mit der Maßgabe, dass mindestens einer der Reste R¹, R², R³, oder R⁴ einen der genannten substituierten oder unsubstituierten Phenylreste bedeutet.

2. Diketopyrrolopyrrol nach Anspruch 1, **dadurch gekennzeichnet, dass** die Reste R¹ und R⁴ gleich sind, und die Reste R² und R³ gleich sind.

3. Diketopyrrolopyrrol nach Anspruch 1 oder 2, **dadurch gekennzeichnet, dass** die Reste R¹, R², R³ und R⁴ unabhängig voneinander Methyl, Ethyl, Phenyl oder Phenyl, das mit 1 oder 2 Substituenten aus der Gruppe Methyl, Ethyl, Methoxy, Ethoxy, CN, F, Cl, S-Methyl, Phenyl oder Benzyl substituiert ist, bedeuten.

4. Diketopyrrolopyrrol nach einem oder mehreren der Ansprüche 1 bis 3, **dadurch gekennzeichnet, dass** R¹ und R⁴ jeweils eine Methyl- oder Ethylgruppe, und R² und R³ jeweils einen gleichen Phenylrest, der unsubstituiert oder durch 1 oder 2 Substituenten aus der Gruppe Methyl, Ethyl, Methoxy, Ethoxy, F, Cl, NO₂, CF₃, Phenyl oder Benzyl substituiert ist, darstellen.

5. Gemisch aus zwei oder mehreren Diketopyrrolopyrrolen nach einem oder mehreren der Ansprüche 1 bis 4.

6. Verfahren zur Herstellung von Diketopyrrolopyrrolen nach einem oder mehreren der Ansprüche 1 bis 5, **dadurch gekennzeichnet, dass** ein Bernsteinsäurediester mit einem Nitril der Formel (II) oder (III), oder mit einem Gemisch von 2, 3 oder 4 verschiedenen Nitrilen der Formel (II) oder (III) 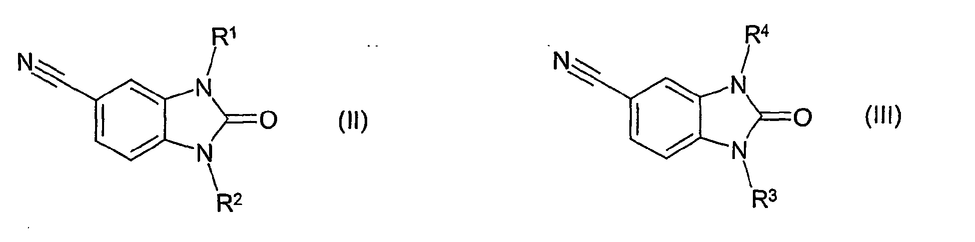 in einem organischen Lösemittel in Gegenwart einer starken Base und anschließender Hydrolyse umgesetzt wird.

7. Verfahren zur Herstellung von Diketopyrrolopyrrolen der Formel (I) nach einem oder mehreren der Ansprüche 1 bis 5, **dadurch gekennzeichnet, dass** ein Ester der Formeln (IV) oder (V) 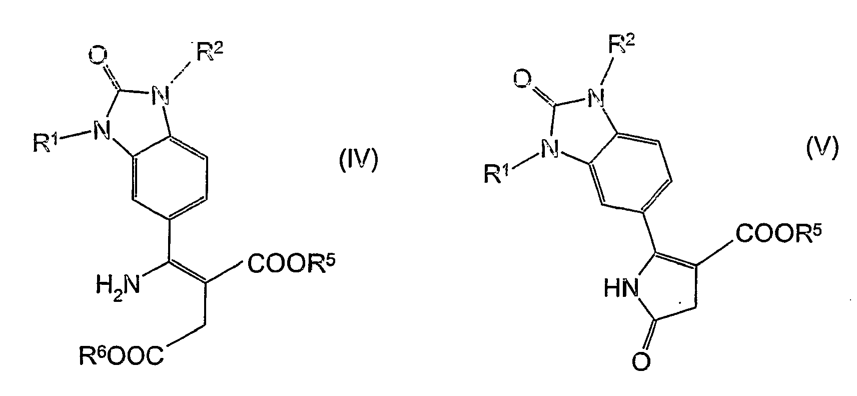 worin R⁵ und R⁶ einen gegebenenfalls substituierten Alkyl- oder Arylrest darstellen,
mit einem Nitril der Formel (III) 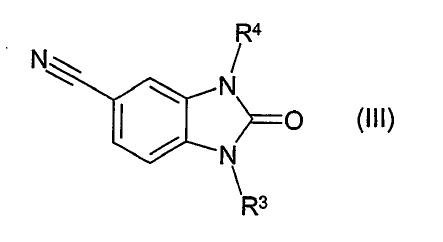 in einem organischen Lösemittel in Gegenwart einer starken Base und anschließender Hydrolyse umgesetzt wird.

8. Verwendung von Diketopyrrolopyrrol nach einem oder mehreren der Ansprüche 1 bis 5 zum Pigmentieren von hochmolekularen organischen Materialien natürlicher oder synthetischer Herkunft, vorzugsweise von Kunststoffen, Harzen, Lacken, Anstrichfarben, oder von elektrophotographischen Tonern und Entwicklern, Farbfiltern sowie von Tinten und Druckfarben.

9. Verwendung nach Anspruch 8, **dadurch gekennzeichnet, dass** die Tinte eine Inkjet-Tinte ist.

10. Verbindung der Formel (IV) oder (V) 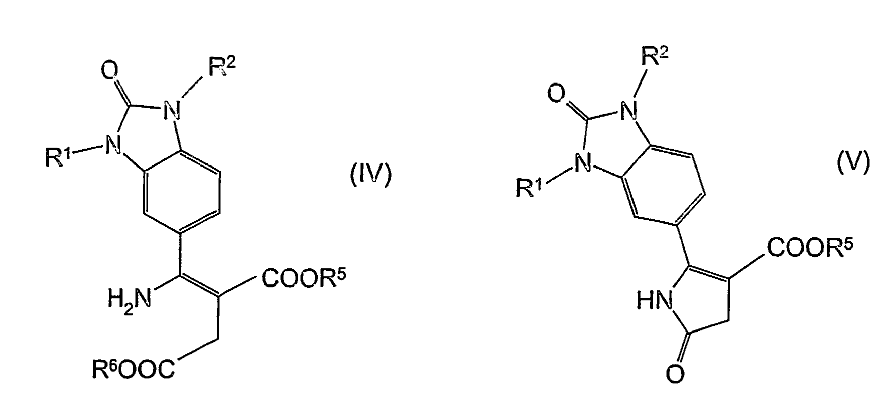 worin R⁵ und R⁶ einen gegebenenfalls substituierten Alkyl- oder Arylrest darstellen und R¹ und R² die in den Ansprüchen 1 bis 4 genannten Bedeutungen haben.

## Claims

1. A diketopyrrolopyrrole of the formula (I) 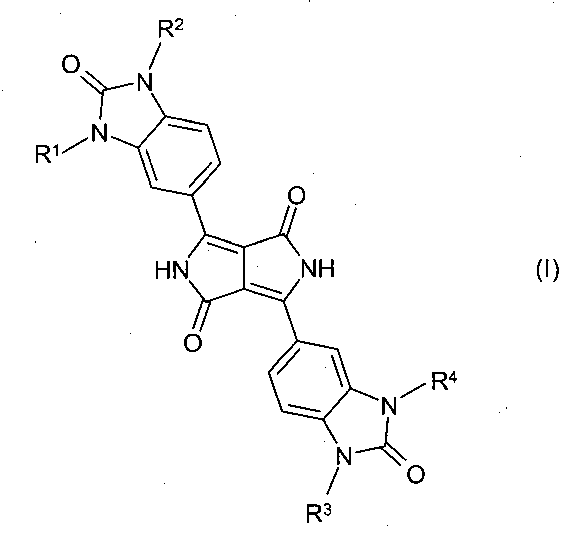 in which
R¹, R², R³ and R⁴ independently of one another are a C₁-C₄ alkyl radical or a substituted or unsubstituted phenyl radical, it being possible for the phenyl radical to be substituted by 1, 2, 3 or 4 substituents from the group C₁-C₄ alkyl, C₁-C₄ alkoxy, CN, F, Cl, Br, NO₂, CF₃, S-C₁-C₄ alkyl, phenyl or (C₁-C₂)alkylenephenyl, with the proviso that at least one of the radicals, R¹, R², R³ or R⁴, is one of the stated substituted or unsubstituted phenyl radicals.

2. A diketopyrrolopyrrole as claimed in claim 1, wherein the radicals R¹ and R⁴ are identical and the radicals R² and R³ are identical.

3. A diketopyrrolopyrrole as claimed in claim 1 or 2, wherein the radicals R¹, R², R³ and R⁴ independently of one another are methyl, ethyl, phenyl or else phenyl substituted by 1 or 2 substituents from the group methyl, ethyl, methoxy, ethoxy, CN, F, Cl, S-methyl, phenyl or benzyl.

4. A diketopyrrolopyrrole as claimed in one or more of claims 1 to 3, wherein R¹ and R⁴ are each a methyl or ethyl group and R² and R³ are each an identical phenyl radical which is unsubstituted or substituted by 1 or 2 substituents from the group methyl, ethyl, methoxy, ethoxy, F, Cl, NO₂, CF₃, phenyl or benzyl.

5. A mixture of two or more diketopyrrolopyrroles as claimed in one or more of claims 1 to 4.

6. A process for preparing diketopyrrolopyrroles as claimed in one or more of claims 1 to 5, which comprises reacting a succinic diester with a nitrile of the formula (II) or (III), or with a mixture of 2, 3 or 4 different nitriles of the formula (II) or (III) 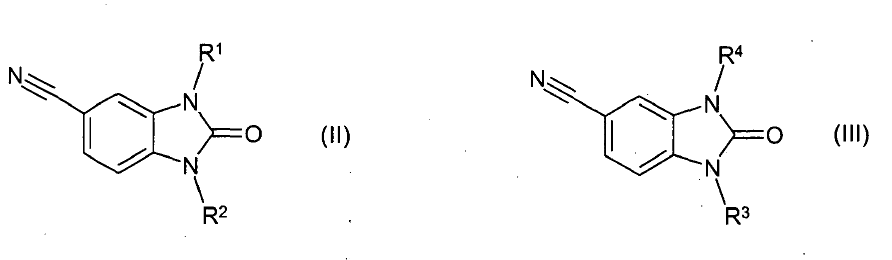 in an organic solvent in the presence of a strong base with subsequent hydrolysis.

7. A process for preparing diketopyrrolopyrroles of the formula (I) as claimed in one or more of claims 1 to 5, which comprises reacting an ester of the formulae (IV) or (V) 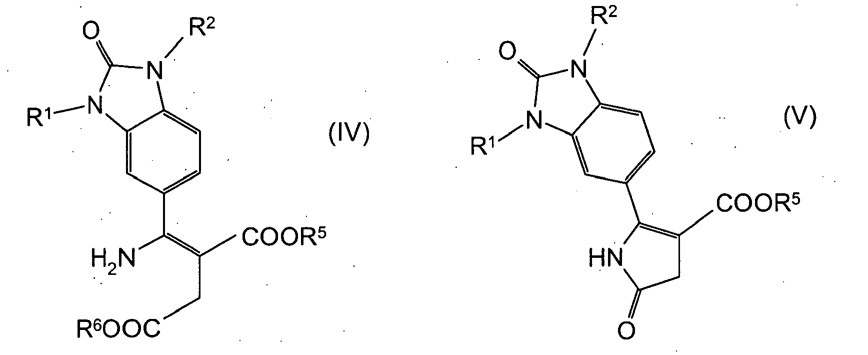 in which R⁵ and R⁶ are an unsubstituted or substituted alkyl or aryl radical,
with a nitrile of the formula (III) 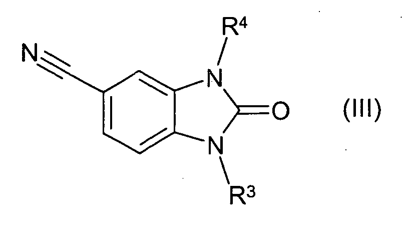 in an organic solvent in the presence of a strong base with subsequent hydrolysis.

8. The use of a diketopyrrolopyrrole as claimed in one or more of claims 1 to 5 for pigmenting high molecular mass organic materials of natural or synthetic origin, preferably plastics, resins, varnishes, paints or electrophotographic toners and developers, color filters and also drawing, writing and printing inks.

9. The use as claimed in claim 8, wherein the ink is an ink-jet ink.

10. A compound of the formula (IV) or (V) 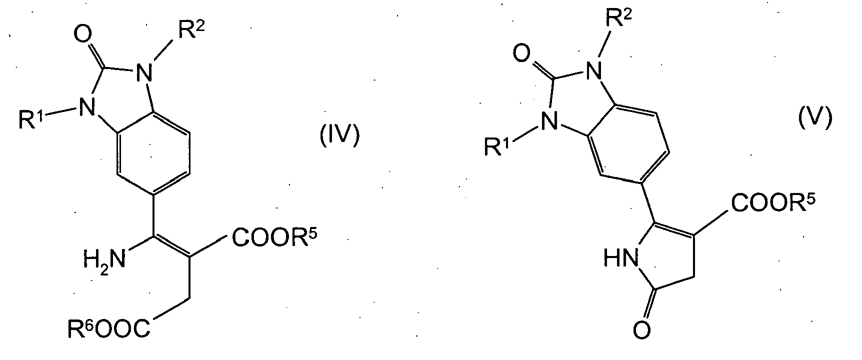 in which R⁵ and R⁶ are an unsubstituted or substituted alkyl or aryl radical and R¹ and R² are as defined in claims 1 to 4.

## Revendications

1. Dicétopyrrolopyrrol de formule (I) 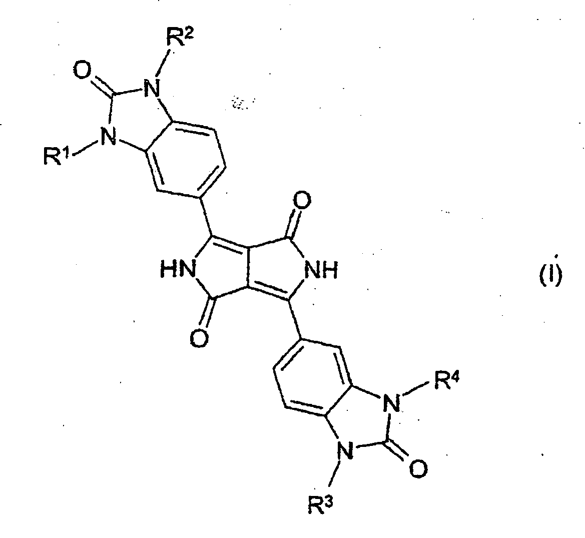 dans laquelle
R¹ R², R³ et R⁴, indépendamment l'un de l'autre, représentent un radical alkyle en C₁ à C₄ ou un radical phényle substitué ou non substitué, le radical phényle pouvant être substitué par 1, 2, 3 ou 4 substituants du groupe alkyle C₁ à C₄, alcoxy en C₁ à C₄, CN, F, Cl, Br, NO₂, CF₃, S-alkyle en C₁ à C₄, phényle ou alkylène phényle (en C₁ à C₂),
à condition qu'au moins l'un des radicaux R¹, R², R³ ou R⁴ représente l'un des radicaux phényle substitués ou non substitués indiqués.

2. Dicétopyrrolopyrrol selon la revendication 1,
**caractérisé en ce que**
les radicaux R¹ et R⁴ sont identiques et les radicaux R² et R³ sont identiques.

3. Dicétopyrrolopyrrol selon la revendication 1 ou la revendication 2,
**caractérisé en ce que** les radicaux R¹, R², R³ et R⁴, indépendamment l'un de l'autre, représentent un groupe méthyle, éthyle, phényle ou phényle substitué par 1 ou 2 substituants du groupe méthyle, éthyle, méthoxy, éthoxy, CN, F, Cl, S-méthyle, phényle ou benzyle.

4. Dicétopyrrolopyrrol selon l'une quelconque ou plusieurs des revendications 1 à 3,
**caractérisé en ce que**
R¹ et R⁴ représentent respectivement un groupe méthyle ou éthyle et R² et R³ respectivement un même radical phényle qui est non substitué ou substitué par 1 ou 2 substituants du groupe méthyle, éthyle, méthoxy, éthoxy, F, Cl, NO₂, CF₃, phényle ou benzyle.

5. Mélange de deux ou plusieurs dicétopyrrolopyrrols selon l'une quelconque ou plusieurs des revendications 1 à 4.

6. Procédé de fabrication de dicétopyrrolopyrrols selon l'une quelconque ou plusieurs des revendications 1 à 5,
**caractérisé en ce qu'**
on convertit un diester de l'acide succinique avec un nitrile de formule (II) ou (III) ou avec un mélange de 2, 3 ou 4 nitriles différents de formule (II) ou (III) 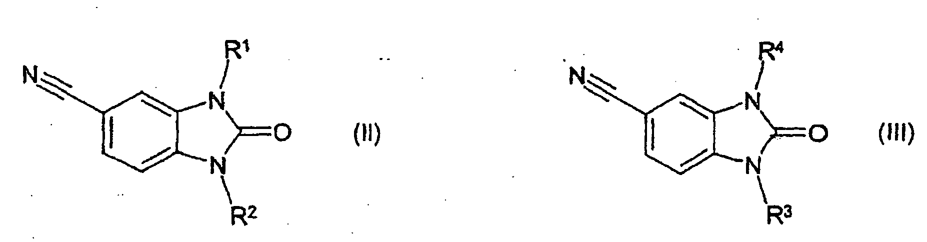 dans un solvant organique en présence d'une base forte et une hydrolyse subséquente.

7. Procédé de fabrication de dicétopyrrolopyrrols selon l'une quelconque ou plusieurs des revendications 1 à 5,
**caractérisé en ce qu'**on convertit un ester de formules (IV) ou (V) 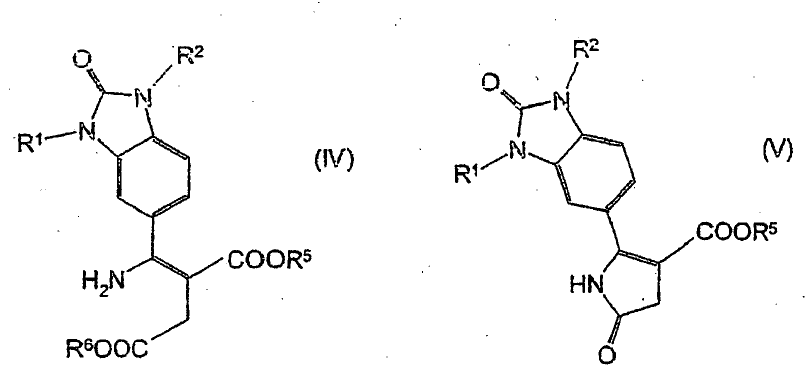 dans laquelle R⁵ et R⁶ représentent un radical alkyle ou aryle éventuellement substitué,
avec un nitrile de formule (III) 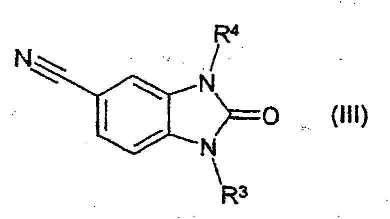 dans un solvant organique en présence d'une base forte et hydrolyse subséquente.

8. Utilisation de dicétopyrrolopyrrol selon l'une quelconque ou plusieurs des revendications 1 à 5 pour pigmenter des matières organiques à haut poids moléculaire d'origine naturelle ou synthétique, de préférence des matières plastiques, des résines, des vernis, des peintures ou des toners et révélateurs électrophotographiques, des filtres colorés ainsi que des encres et encres d'imprimerie.

9. Utilisation selon la revendication 8,
**caractérisé en ce que**
l'encre est une encre pour impression à jet d'encre.

10. Composé de formule (IV) ou (V) 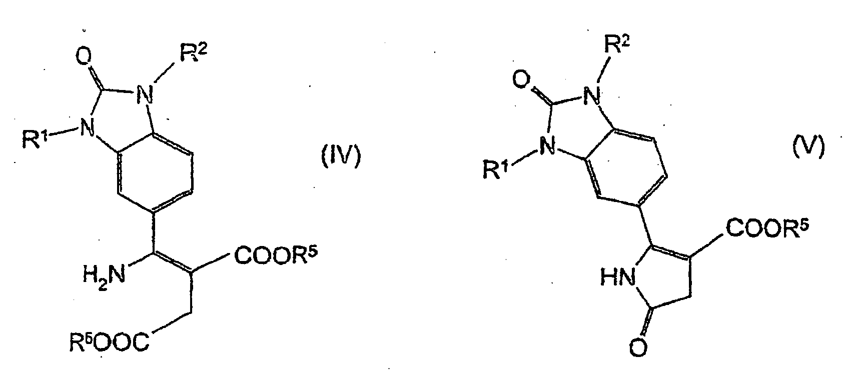 dans laquelle R⁵ et R⁶ représentent un radical alkyle ou aryle le cas échéant substitué et R¹ et R² ont les significations indiquées dans les revendications 1 à 4.
